# EUROPÄISCHE PATENTSCHRIFT

(11) **EP 2 776 817 B1**
(45) Veröffentlichungstag und Bekanntmachung des Hinweises auf die Patenterteilung: **01.06.2022**
(21) Anmeldenummer: 12743726.7
(22) Anmeldetag: 03.08.2012
(51) Int. Cl.: G01N 21/90, G01N 33/14

(54) **INSPEKTION UND RÜCKFÜHRUNG VON BEHÄLTERN**
INSPECTION AND RECYCLING OF CONTAINERS
CONTRÔLE ET RETOUR DE RÉCIPIENTS

(30) Priorität: 10.11.2011 DE 102011086099
(43) Veröffentlichungstag der Anmeldung: 17.09.2014
(73) Patentinhaber: Krones AG, 93073 Neutraubling (DE)
(72) Erfinder: KOLB, Herbert, 93356 Teugn (DE)
(74) Vertreter: Grünecker Patent- und Rechtsanwälte PartG mbB
(86) Internationale Anmeldenummer: PCT/EP2012/065274
(87) Internationale Veröffentlichungsnummer: WO 2013/068136

(56) Entgegenhaltungen:
- EP-A1- 2 383 567
- EP-A2- 1 939 630
- EP-A2- 2 369 328
- US-A- 4 915 237

## Beschreibung

### Stand der Technik

Die Erfindung betrifft eine Anlage und ein Verfahren zur Inspektion von befüllten Behältnissen, beispielsweise Flaschen, deren Inhalt beispielsweise mit CO2 versetzt ist.

Aus der DE 102 57 749 B4 ist eine Inspektionsmaschine bekannt, welche mit einem Produkt befüllte und verschlossene Behälter auf Verunreinigungen, z. B. Glassplitter untersucht. Dabei wird der Behälter zunächst in Rotation um die eigene Achse versetzt, bis das Produkt, die Flüssigkeit, der Rotation (teilweise) folgt. Anschließend wird der Behälter gestoppt, die Flüssigkeit rotiert weiter. In diesem Zustand wird der Behälter beleuchtet und mittels einer Kamera beobachtet. In diesem sogenannten Spin-Stop-Verfahren ist die Bewegung der Flüssigkeit im Kamerabild nicht zu sehen, die Verunreinigung und dessen Bewegung jedoch schon. Verunreinigungen können sein: Glassplitter oder andere Feststoffe, die im Produkt nicht erwünscht sind, insbesondere Glassplitter sind für den Endverbraucher gefährlich.

Aus der WO9714956A1 ist bekannt, dass in CO2-haltigen Produkten, wie Bier, Cola, Limonade und andere, bei der Rotation um die eigene Achse je nach CO2-Gehalt und Lösungsgrad des CO2's im Produkt, feine Gasbläschen entstehen können. Diese bewegen sich ebenso wie mögliche Fremdkörper im Produkt, bzw. in einer Flasche. Eine Unterscheidung von Gasbläschen und Fremdkörpern nach oben genanntem Spin-Stop-Verfahren ist in solchen Fällen kaum möglich.

Aus den Dokumenten EP 2 383 567 A1, EP 2 369 328 A2 und EP 1 939 630 A2 sind weitere Inspektionsvorrichtungen bekannt.

Die Chance einer irrtümlichen Ausleitung/Aussortierung von Flaschen aufgrund von Gasbläschen und nicht aufgrund von Fremdkörpern ist gegeben. Minimieren könnte man dies durch eine lange Verweilzeit zwischen Füllen und Inspizieren, was aber lange Transportwege oder sehr große Massentransporteure erfordert. Diese technische Nachrüstung ist zudem aufwendig und erhöht die Produktionskosten.

Die Bildung von unerwünschten Gasbläschen in CO2-haltige Produkten kann nicht nur durch ein Inspektionsverfahren selbst, sondern auch durch verschiedene andere Störungen eines CO2-haltigen Produktes im Laufe des Produktionsweges verursacht werden.

### Aufgabe

Es ist somit Aufgabe der Erfindung eine Vorrichtung und ein Verfahren anzugeben, welches die Fehlerquote bei der Inspektion/Untersuchung von Behältern, welche mit CO2-haltigen Produkten, wie beispielsweise Bier, Cola, oder Limonade befüllt sind, zu verbessern/minimieren.

### Lösung

Dies wird erfindungsgemäß durch ein Verfahren nach Anspruch 1 und eine Vorrichtung nach Anspruch 10 erreicht. Vorteilhafte Ausführungsformen und Weiterbildungen sind Gegenstand der Unteransprüche.

Der Vollständigkeit halber sei an dieser Stelle erwähnt, dass wenn im Folgenden von Behältern die Rede ist, Behälter gemeint sind, die befüllt sind, insbesondere befüllt mit CO2-haltigen Produkten.

Dabei können bei der Untersuchung von befüllten Behältern, die mit CO2-haltigen Produkten, wie beispielsweise Bier oder Limonade befüllt sind, auf Verunreinigungen, wie beispielsweise Glassplitter, die Behälter auf Gasbläschen im Produkt/im Behälter und/oder auf einen Gasnebel im Produkt/Behälter untersucht werden. Führt diese Untersuchung zur Feststellung von Gasbläschen im Produkt/Behälter und/oder Gasnebel im Produkt/Behälter, können diese Behälter zunächst aussortiert werden, um mindestens ein erneutes Mal untersucht zu werden, bevor sie entweder unbeanstandet die erneute Untersuchung auf Verunreinigungen passieren, oder endgültig aussortiert bzw. aus dem Produktionsprozess entfernt werden.

Dies hat den Vorteil, dass Behälter, die zwar Gasbläschen im Produkt aufweisen oder einen Gasnebel, aber nicht verunreinigt sind, zu einem späteren Zeitpunkt erneut untersucht werden können. Während der Zeit zwischen zwei Untersuchungen, können evtl. vorhandene Gasbläschen oder Gasnebel im Behälter verschwinden, in dem sie sich wieder im Produkt lösen. Auf diese Weise können Behälter, die nicht verunreinigt sind, sondern bei einer ersten Untersuchung nur Gasbläschen im Produkt und/oder einen Gasnebel aufgewiesen haben, bei einer erneuten Untersuchung, als beanstandungsfrei / verunreinigungsfrei befunden werden wieder in den Produktionsprozess zurückgeführt werden. Die Fehlerquote von fälschlicherweise aus dem Produktionsprozess ausgeleiteter bzw. entfernter Behälter, welche nicht verunreinigt sind, kann somit im Vergleich zu Verfahren, welche beispielsweise bei Detektion von Gasbläschen im Produkt, solche Behälter ohne erneute Untersuchung aus dem Produktionsprozess entfernen, reduziert werden.

Behälter, bei denen bei der ersten Untersuchung auf Verunreinigungen, Verunreinigungen, wie z.B. Glassplitter, festgestellt wurden, können natürlich nach der ersten Untersuchung ausgeleitet/aussortiert und aus dem weiteren Produktionsprozess endgültig entfernt werden, während Behälter, bei denen keine Verunreinigungen und keine Gasbläschen im Produkt und/oder Gasnebel detektiert wurden, unbeanstandet die Untersuchung passieren und weiteren Schritten im Produktionsprozess zugeführt werden können.

Die Untersuchung von Behältern, die mit CO2-haltigen Produkten befüllt sind, auf Verunreinigung, wie beispielsweise Glasscherben, kann dabei mittels optischer/infrarot/photometrischer bzw. lichtmikroskopischer Verfahren, wie beispielsweise Hellfeld- und/oder Dunkelfeldmikroskopie, Phasenkontrast-, Interferenzkontrast-, Fluoreszenz-, Polarisations- und Konfokalmikroskopie, im Rahmen eines Eingangs erwähnten und in der WO97/14956A1 beschriebenen Spin-Stop-Verfahrens, durchgeführt werden. Bei den Spin-Stop-Verfahren wird der Behälter in Rotation versetzt, so dass sich der Behälterinhalt zu drehen beginnt. Bei dann angehaltenem Behälter werden nacheinander zwei, drei oder mehr Bildaufnahmen gemacht, bei denen sich der Behälterinhalt zwischen jeweils zwei Bildaufnahmen bewegt hat, der Behälter jedoch nicht (in Bezug auf die Kamera). Durch Vergleich der beiden Bilder kann auf sich bewegende Verunreinigungen bzw. Gasbläschen geschlossen werden. Das Spin-Stop-Vefahren kann durchgeführt werden, während sich die Behälter auf einem Karussell befinden, das mehrere Aufnahmeplätze für Behälter aufweist, an denen jeweils ein Behälter in Drehung versetzt werden kann.

Die Unterscheidung zwischen Verunreinigungen, Gasbläschen im Produkt/im Behälter kann dabei z.B. auf einer Analyse eines Farb-/Graustufenpektrums hinsichtlich Kontrast und/oder Helligkeit und/oder Farb-/Graustufenwert in einer Kameraaufnahme beruhen. So können z.B. Schwellenwerte in den Unterschieden und/oder Verhältnissen von, Helligkeit und/oder Kontrast zwischen einem Objekt, beispielsweise eine Verunreinigung (z.B. eine Glasscherbe), ein Glasbläschen und dem Produkt, d.h. dem vorgesehenen Behälterinhalt, definiert werden.

Beispielsweise kann die Identifikation/Klassifikation verschiedener Objekte im Behälter über das Unter-bzw. Überschreiten eines Helligkeitsschwellenwertes um z.B. mindestens 1, 10, 100 % in Bezug auf den Unterschied der Helligkeit von Objekten, z.B. Objekten/Bereichen in den Größenordnungen, mindestens 0,01, 0,1, 1, 10, 20 mm (oder größer), im Vergleich zum vorgesehenen Produkt / Behälterinhalt, definiert werden.

Die Position detektierter Objekte innerhalb des Behälters, sowie deren Anzahl und/oder räumliche Ausdehnungen, kann ebenso/zusätzlich zur Festlegung/Identifikation der Objekte im Behälter dienen. Beispielsweise kann eine Mindestanzahl von Objekten von 10, 100 oder 1000 mit räumlichen Ausdehnungsmaßen z.B. in den Größenordnungen von mindestens 0,01, 0,1, 1, 10, 20 mm und/oder Aufenthaltspositionen von Objekten im Behälter innerhalb von vorbestimmten Bildbereichen, dazu verwendet werden Objekte, als Gasbläschen und/oder Verunreinigungen zu klassifizieren.

Bei einer großen Zahl von Gasbläschen können diese evtl. auch nicht mehr einzeln identifiziert werden, da sie sich insgesamt als nebelartig darstellen. Das Vorhandensein eines solchen Gasnebels kann beispielsweise dadurch definiert werden, dass auf einem Kamerabild, bzw. auf einem von einem oder mehreren Kamerabildern abgeleiteten Bild, beispielsweise im Zentrum des Behälters, ausgedehnte Bereiche (mit räumlichen Ausdehnungen von z.B. mindestens 0,1, 0,5, 1,0, 2,0 cm) einen Farb- und/oder Grauwert- und/oder Helligkeits- und/oder Kontrastschwellwert in Bezug zum vorgesehenen Produkt / Behälterinhalt, um z.B. mindestens 1, 10, 100 % unter- bzw. überschreiten, und/oder besagte ausgedehnte Bereiche eine Variation/Variationen von weniger als maximal 40, 30, 10 % in Bezug auf den Mittelwert oder Median der Verteilung von Farb- und/oder Grauwerte- und/oder Helligkeits- und/oder Kontrastwerte innerhalb besagter ausgedehnter Bereiche aufweisen, und/oder die ausgedehnten Bereiche mit vom Behälterprodukt in oben beschriebener Weise abweichenden Werten für Farb- und/oder Grauwert- und/oder Helligkeits- und/oder Kontrastwert, in Ihrer Form der Behälterinnenkontur folgen.

Die Detektion eines Gasnebels im Produkt kann an einem einzelnen, aufgenommenen Bild erfolgen, ohne dass hierfür die Differenz von zwei Bildern aus dem Spin-Stop-Verfahren notwendig wäre. Für die Detektion von einzelnen Verunreinigungen oder Gasbläschen wird besser ein Differenzbild aus beispielsweise einem Spin-Stop-Verfahren ausgewertet.

Anstatt von Schwellenwerten bezüglich Kontrast und/oder Helligkeit und/oder Farb-/Graustufenwert in einer Kameraaufnahme, bzw. in einem von einer/mehreren Kameraufnahmen abgeleiteten Bild, zur Klassifizierung von verschiedenen Objekten, bzw. verschiedenen Bereichen in einem befüllten Behälter, können auch Wertebereiche der Werte für Kontrast und/oder Helligkeit und/oder Farb-/Graustufen verwendet werden.

Auch ist denkbar, dass eine Klassifikation/Identifikation von verschiedenen Objekten, bzw. verschiedenen Bereichen in einem befüllten Behälter, über Infrarotaufnahmen erfolgt, aufgrund verschiedener Temperaturen der Objekte/ausgedehnten Bereiche im befüllten Behälter. Auch hier lassen sich Schwellwerte und Wertebereich definieren, beispielsweise Schwellwerte für Temperaturunterschiede von mindestens 0,1, 0,5, 10, 20°C.

Ebenso, bzw. zusätzlich, können Objekte im Behälter aufgrund Ihrer Form klassifiziert werden.

Beispielsweise können Objekte im Behälter als Gasbläschen identifiziert werden, wenn die numerische Exzentrizität ihrer projizierten Kontur in der Kameraaufnahme, bzw. in einem von einer/mehreren Kameraaufnahmen abgeleiteten Bild, nicht größer ist als beispielsweise 0,1, 0,2, 0,3.

Nach einer Untersuchung von Behältern, welche mit CO2-haltigen Produkten, wie beispielsweise Bier, Cola, oder Limonade befüllt sind, und bei denen Gasbläschen im Produkt/Behälter und/oder Verunreinigungen im Behälter detektiert wurden, können diese markiert werden, beispielsweise mit einem Aufdruck oder einer Etikettierung. Somit kann ein eventuelles dauerhaftes Kreisen von beanstandeten Behältern innerhalb des Produktionsablaufes vermieden werden. Bei der Detektion von Verunreinigungen können die Behälter endgültig ausgeleitet werden (auch ohne Markierung oder Etikettierung).

Die Figuren stellen beispielhaft dar:
**Fig.1****:** Anlage zur Untersuchung von befüllten Behältern.
**Fig.2****:** Befüllter Behälter.
**Fig.3****:** Bildauswertungsdiagramm.

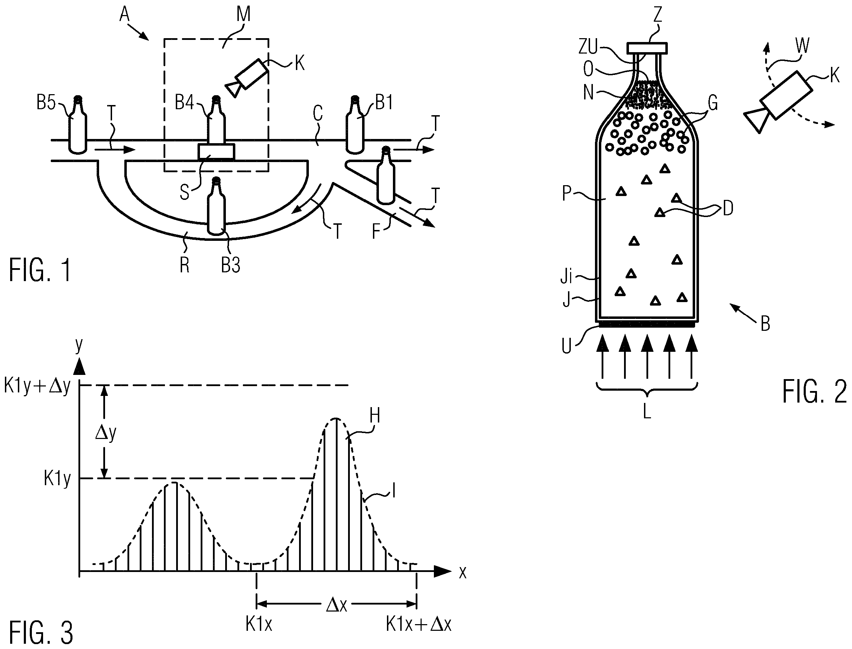

Fig.1 zeigt beispielhaft eine Anlage A zur Untersuchung von befüllten Behältern, die mit CO2-haltigen Produkten, wie beispielsweise Bier oder Limonade befüllt sind, auf Verunreinigungen, wie beispielsweise Glassplitter. Eine Messeinrichtung M kann dabei einen Behälter mit einer Kamera K untersuchen, wobei der Behälter durch die Behälterbehandlungseinheit S beispielsweise ein Spin-Stop-Verfahren durchläuft. Je nach Resultat der Untersuchung kann die Messeinrichtung einen untersuchten Behälter an eine der drei Behälterführungen wie z.B. Behälterbeförderungsbänder R, F oder C weiterleiten. Anstelle von Beförderungsbändern können auch andere Transportmöglichkeiten vorgesehen sein, beispielsweise solche mit bewegbaren Greifern zum Greifen der Behälter.

Ein unbeanstandeter Behälter, bei dem weder Verunreinigungen, noch Glasbläschen im Produkt noch Gasnebel im Behälter festgestellt wurden, kann z.B. auf Förderband C die Messeinrichtung M verlassen und zum nächsten Produktionsschritt weitergeleitet werden.

Behälter, bei denen Verunreinigungen, wie z.B. Glassscherben, detektiert wurden, und/oder Behälter bei denen zum wiederholten Mal Glasbläschen im Produkt oder Gasnebel im Behälter festgestellt wurde, können über Förderband F aus dem Produktionsprozess ausgeleitet/entfernt werden.

Bei der Untersuchung von den Behältern gibt es beispielsweise drei verschiedene Detektionsergebnisse wie z.B. "verunreinigungsfrei", "verunreinigt" und "Bläschen und/oder Nebel vorhanden". Je nach Detektionsergebnis werden die Behälter entsprechend verschieden weitergeleitet.

Behälter, bei denen bei einer ersten Untersuchung Glasbläschen im Produkt und/oder Gasnebel im Behälter festgestellt wurden, können über Förderband R zu einer erneuten Untersuchung durch Messeinrichtung M zurückgeführt werden. Allerdings ist auch denkbar, dass Förderband R solche Behälter auch zu einer anderen von M verschiedenen alternativen Messeinrichtung leiten kann.

Ebenso ist es möglich, dass die Anlage A zur Untersuchung von befüllten Behältern eine Parkstation aufweist, in der befüllte Behälter, welche aussortiert wurden, um erneut auf Verunreinigungen untersucht werden zu können, vor der erneuten Untersuchung auf Verunreinigungen, geparkt werden, beispielsweise für Zeiträume von mindestens 0,1, 1, 10, 100 h. Die Zuführung zur Parkstation kann beispielsweise über Förderband R, oder ein weiteres zusätzliches Förderband (oder sonstige Behältertransportmittel) erfolgen. Dies hat den Vorteil, dass die Zeitspanne zwischen zwei Untersuchungen desselben befüllten Behälters auf Verunreinigungen, reguliert werden kann, unabhängig bzw. zusätzlich von der Transportgeschwindigkeit der Förderbänder/eines Förderbandes.

Fig.2. zeigt beispielhaft einen Behälter B, der mit einem Produkt P befüllt und mit einem Verschluss Z verschlossen ist. Es ist ein Gasnebel N dargestellt, der sich aus vielen Gasbläschen zusammensetzt, die nicht mehr einzeln von einem Kamerabild aufgelöst werden können. Auch sind beispielhaft Gasbläschen G sowie Verunreinigungen D gekennzeichnet.

Durch den Behälterboden dringt beispielhaft Licht L in den Behälter B ein, um dessen Inhalt untersuchen zu können. Das Licht L kann aber auch seitlich oder von oben auf den Behälter treffen. Eine beispielhaft seitlich angeordnete Kamera K, welche sich zudem z.B. entlang der Bewegungsrichtung W bewegen kann, kann Aufnahmen vom Behälter und/oder seinem Inhalt machen.

Die Innenseite Ji der Behälterwand J verbunden mit der unteren Seite ZU des Verschlusses definiert die Behälterinnenkontur.

Fig. 3 zeigt ein Diagramm, welches beispielhaft verschiedene Auswertungsmöglichkeiten von beispielweise einer Kamera gemachten/abgeleiteten Bilder eines auf Verunreinigungen zu untersuchen Behälters veranschaulicht. So kann z.B. die X-Achse für verschieden Graustufen in einem Untersuchungsbild stehen und die Y-Achse für die Häufigkeiten der Graustufen. Dabei kann z.B. die Häufigkeitsverteilung der Graustufen als Histogramm H dargestellt/verwendet werden, oder z.B. eine Interpolation I des Histogramms H. Mittels Definition von Schwellenwerten wie K1x und/oder K1y, bzw. Wertebereichen K1x+Δx und/oder K1y+Δy können so beispielsweise Graustufenschwellwerte und/oder Graustufenwertebereiche definiert werden, um so verschiedene Objekte im Behälter, wie z.B. Gasnebel zu identifizieren/klassifizieren. Analog kann die X-Achse für Farbstufen, Kontrast-/Helligkeitsstufen, Wellenlänge, Frequenz, usw. stehen, und die Y-Achse unter anderem für Intensität, spektrale Leistungsdichte, usw. Ebenso können die Schwellwerte K1xK1y bzw. die Wertebereiche K1x+Δx und/oder K1y+Δy für genannte Größen stehen.

Es werden die folgenden Bezugszeichen verwendet :
**A** Vorrichtung/Anlage zur Inspektion/Untersuchung von Behältern, welche mit CO2-haltigen Produkten befüllt sind.
**B, B1, B2, B3, B4, B5** befüllte Behälter/Behältnisse.
**C** Behälterbeförderungsband/Förderband zur Weiterleitung unbeanstandeter Behälter.
**F** Behälterbeförderungsband/Förderband zur Ausleitung beanstandeter Behälter aus dem Produktionsprozess.
**R** Behälterbeförderungsband/Förderband/Rückführband zur Rückführung von Behälter zu einer erneuten Untersuchung auf Verunreinigungen.
**S** Behälterbehandlungseinheit für Spin-Stop-Verfahren.
**T** Behälterbeförderungsrichtung.
**K** Kamera, beispielsweise Infrarotkamera, Videokamera, Fotokamera, usw.
**N** Gasnebel.
**G** Gasbläschen.
**D** Verunreinigungen, z.B. Glassscherben, Glassplitter.
**P** Produkt / vorgesehener Behälterinhalt.
**Z** Behälterverschluss.
**ZU** Unterseite von Behälterverschluss.
**O** Oberfläche des Produktes / vorgesehener Behälterinhaltes.
**L** Licht.
**U** Behälterboden.
**J** Behälterwand.
**Ji** Innenseite von Behälterwand.
**W** Freiheitsgrad/Bewegungsrichtung für Kamera.
**H** Histogramm.
**I** Interpolationskurve des Histogramms H.
**X** Ordinate eines Bildauswertungsdiagramms, beispielsweise Graustufen, Farbstufen, Kontrast-/Helligkeitsstufen, Wellenlänge, Frequenz, usw.
**Y** Abszisse eines Bildauswertungsdiagramms, beispielsweise, Häufigkeit, Intensität. spektrale Leistungsdichte, usw.
**K1x** Schwellenwert für Ordinatengröße.
**K1y** Schwellenwert für Abszissengröße.
Δ**x**, Δ**y** gewünschter Wertebereich für Analyse einer Ordinaten-/Abszissengröße.

## Patentansprüche

1. Verfahren zur Untersuchung von befüllten Behältern (B, B1, B2, B3, B4, B5), die mit CO2-haltigen Produkten, wie beispielsweise Bier oder Limonade befüllt sind, auf Verunreinigungen, wie beispielsweise Glassplitter, welches beinhaltet, dass ein Behälter auf Gasbläschen (G) im Produkt (P) / im Behälter (B) und/oder Gasnebel (N) im Produkt (P) / Behälter (B) untersucht wird, beispielsweise mit einer Kamera, **dadurch gekennzeichnet, dass** der Behälter bei Feststellung von Gasbläschen (G) im Produkt (P) / Behälter (B) und/oder Feststellung von Gasnebel (N) im Produkt (P) / Behälter (B) aussortiert wird und der Behälter zu einem späteren Zeitpunkt erneut auf Verunreinigungen untersucht wird.

2. Verfahren nach Anspruch 1, bei dem die Untersuchung der befüllten Behälter (B, B1, B2, B3, B4, B5) auf Verunreinigung ein Spin-Stop-Verfahren beinhaltet.

3. Verfahren nach einem der vorigen Ansprüche, welches beinhaltet, dass die Feststellung von Gasbläschen (G) im Produkt (P) / Behälter (B) darüber erfolgt, dass eine Mindestanzahl, beispielsweise mindestens, 10, 100 oder 1000 von Objekten im Produkt (P) bzw. Behälter (B) mit räumlichen Ausdehnungsmaßen z.B. in den Größenordnungen von mindestens 0,01, 0,1, 1, 10, 20 mm und/oder besagte Objekte an vorbestimmten Aufenthaltspositionen detektiert werden.

4. Verfahren nach einem der vorigen Ansprüche, welches beinhaltet, dass die Feststellung von Gasnebel (N) im Produkt (P) / Behälter (B) darüber erfolgt, dass auf einem Kamerabild vom Behälter (B) bzw. auf einem von einem oder mehreren Kamerabildern abgeleiteten Bild ausgedehnte Bereiche, beispielsweise mit räumlichen Ausdehnungen von z.B. mindestens 0,1, 0,5, 1,0, 2,0 cm, einen Farb- und/oder Grauwert- und/oder Helligkeits- und/oder Kontrastschwellwert in Bezug zum vorgesehenen Produkt (P) / Behälterinhalt, um z.B. mindestens 1, 10, 100 % unter- bzw. überschreiten,
und/oder die Feststellung von Gasnebel (N) im Produkt (P) / Behälter (B) darüber erfolgt, dass besagte ausgedehnte Bereiche eine Variation/Variationen von weniger als beispielsweise maximal 40, 30, 10 % in Bezug auf den Mittelwert oder Median der Verteilung von Farb- und/oder Grauwerte- und/oder Helligkeits- und/oder Kontrastwerte innerhalb besagter ausgedehnter Bereiche aufweisen und/oder die ausgedehnten Bereiche, mit vom Behälterprodukt (P) in oben beschriebener Weise abweichenden Werten für Farb- und/oder Grauwert- und/oder Helligkeits- und/oder Kontrastwert in Ihrer Form, der Behälterinnenkontur folgen.

5. Verfahren nach einem der vorigen Ansprüche, welches beinhaltet, dass Objekte im Behälter (B) aufgrund Ihrer Form klassifiziert werden, z.B. können Objekte im Behälter (B) als Gasbläschen (G) identifiziert werden, wenn die numerische Exzentrizität ihrer projizierten Kontur in der Kameraaufnahme, bzw. in einem von einer/mehreren Kameraaufnahmen abgeleiteten Bild, nicht größer ist als beispielsweise mindestens 0,1, 0,2, 0,3.

6. Verfahren nach einem der vorigen Ansprüche, welches beinhaltet, dass die Feststellung von Gasbläschen (G) im Produkt (P) und/oder von Gasnebel (N) im Behälter (B), und/oder Verunreinigungen im Behälter (B), darüber erfolgt, dass Schwellwerte für Temperaturunterschiede, z.B. von mindestens 0,1, 0,5, 10, 20°C zur Unterscheidung/Klassifizierung der verschiedenen Objekte/ausgedehnter Bereiche im Behälter (B) verwendet werden.

7. Verfahren nach einem der vorigen Ansprüche, welches beinhaltet, dass mit CO2-haltigen Produkten befüllte Behälter (B), bei denen bei einer ersten Untersuchung eine Verunreinigung festgestellt wurde, markiert werden, beispielsweise mit einem Aufdruck/einer Etikette, und/oder aus dem Produktionsprozess ausgeleitet werden, und mit CO2-haltigen Produkten befüllte Behälter (B), bei denen weder Verunreinigungen noch Gasbläschen (G) im Produkt (P) / Behälter (B) und/oder Gasnebel (N) im Produkt (P) / Behälter (B) festgestellt wurden, unbeanstandet zum nächsten Produktionsprozess weitergeleitet werden.

8. Verfahren nach einem der vorigen Ansprüche, welches beinhaltet, dass mit CO2-haltigen Produkten befüllte Behälter (B) aus dem Produktionsprozess endgültig ausgeleitet werden, wenn bei einer erneuten Untersuchung auf Verunreinigungen, Gasbläschen (G) im Produkt (P) / Behälter (B) und/oder Gasnebel (N) im Produkt (P) / Behälter (B) festgestellt werden.

9. Verfahren nach einem der vorigen Ansprüche, welches beinhaltet, dass befüllte Behälter (B), welche aussortiert wurden, um erneut auf Verunreinigungen untersucht werden zu können, vor der erneuten Untersuchung auf Verunreinigungen, geparkt werden, beispielsweise für Zeiträume von mindestens 0,1, 1, 10, 100 h.

10. Vorrichtung (A) zur Untersuchung von befüllten Behältern (B, B1, B2, B3, B4, B5), die mit CO2-haltigen Produkten, wie beispielsweise Bier oder Limonade befüllt sind, auf Verunreinigungen, wie beispielsweise Glassplitter, mit einer Messeinrichtung (M), welche beispielsweise mit einer Kamera , einen zu untersuchenden Behälter (B) auf Gasbläschen (G) im Produkt (P) / Behälter (B) und/oder auf Gasnebel (N) im Produkt (P) / Behälter (B) und auf Verunreinigungen im Produkt (P) /Behälter (B) untersuchen kann, **dadurch gekennzeichnet, dass** die Vorrichtung dazu konfiguriert ist, bei Feststellung von Gasbläschen (G) im Produkt (P) / im Behälter (B) und/oder Feststellung von Gasnebel (N) im Produkt (P) / Behälter (B), den Behälter (B) auszusortieren, und den Behälter mit einer Behälterführung (R) für eine erneute Untersuchung auf Verunreinigungen zu einem späteren Zeitpunkt mittels der Messeinrichtung (M) zur Messeinrichtung (M) oder zu einer anderen alternativen Messeinrichtung zu führen.

11. Vorrichtung nach Anspruch 10, **dadurch gekennzeichnet, dass** die Vorrichtung (A) zur Untersuchung von befüllten Behältern (B) eine Behälterbehandlungseinheit (S) für ein Spin-Stop-Verfahren aufweist.

12. Vorrichtung nach einem der vorherigen Ansprüchen 10 bis 11, **dadurch gekennzeichnet, dass** die Vorrichtung (A) zur Untersuchung von befüllten Behältern (B) eine Behälterparkstation aufweist, welche befüllte Behälter (B) aufnehmen kann.

13. Vorrichtung nach einem der vorherigen Ansprüchen 10 bis 12, **dadurch gekennzeichnet, dass** die Messeinrichtung (M) eine Infrarotkamera (K) aufweist.

## Claims

1. A method for examining filled containers (B, B1, B2, B3, B4, B5), which are filled with CO₂-containing products such as beer or lemonade, for contaminants such as glass fragments, said method comprising the steps of examining, e.g. by means of a camera, a container for gas bubbles (G) in the product (P) / in the container (B) and/or gas mists (N) in the product (P) / container (B), and sorting out the container if gas bubbles are determined in the product (P) / container (B) and/or if gas mists (N) are determined in the product (P) / container (B), and re-examining the container later on.

2. The method according to claim 1, wherein the examination of the filled containers (B, B1, B2, B3, B4, B5) includes a spin-stop process.

3. The method according to one of the preceding claims, comprising that gas bubbles (G) in the product (P) / container (B) are determined in that a minimum number of objects, e.g. at least 10, 100 or 1000, is detected in the product (P) and/or container (B), with spatial dimensions e.g. in orders of magnitude of at least 0.01, 0.1, 1, 10, 20 mm, and/or that said objects are detected at predetermined dwell positions.

4. The method according to one of the preceding claims, comprising that gas mists (N) in the product (P) / container (B) are determined in that on a camera image of the container or on an image derived from one or from a plurality of camera images, large areas, e.g. areas with spatial dimensions of e.g. at least 0.1, 0.5, 1.0, 2.0 cm, underrun or exceed a colour and/or grey value and/or brightness and/or contrast threshold value with respect to the provided product (P) / container (B) content by e.g. at least 1, 10, 100%,
and/or that gas mists (N) in the product (P) /container (B) are determined in that said large areas exhibit a variation/variations of less than a maximum of 40, 30, 10 % with respect to the mean value or median of the distribution of colour and/or grey values and/or brightness and/or contrast values within said large areas, and/or the large areas with values for colour and/or grey values and/or brightness and/or contrast values deviating from the container product (P) in above-described way follow the inner contour of the container as regards their shape.

5. The method according to one of the preceding claims, comprising that objects in the container (B) are classified on the basis of their shape, objects in the container(B) being e.g. classifiable as gas bubbles (G), if the numerical eccentricity of their projected contour in the camera image, or in an image derived from one / a plurality of camera images, is not larger than e.g. at least 0.1, 0.2, 0.3.

6. The method according to one of the preceding claims, comprising that gas bubbles (G) in the product (P) and/or gas mists (N) in the container (B) and/or contaminants in the container (B) are determined in that threshold values for temperature differences of e.g. at least 0.1, 0.5, 10, 20°C are used for differentiating between / classifying the various objects / large areas in the container (B).

7. The method according to one of the preceding claims, comprising that containers (B) which are filled with CO₂-containing products and in which contaminants have been determined during a first examination are marked, e.g. by means of an imprint / a label, and/or discharged from the production process, and containers (B) which are filled with CO₂- containing products and in which neither contaminants nor gas bubbles (G) nor gas mists (N) have been determined in the product (P) / container (B) are advanced to the next production step without being objected to.

8. The method according to one of the preceding claims, comprising that containers (B) which are filled with CO₂-containing products are discharged from the production process once and for all, if contaminants, gas bubbles (G) in the product (P) / container (B) and/or gas mists (N) in the product (P) / container (B) are determined during renewed examination.

9. The method according to one of the preceding claims, comprising that filled containers (B), which have been sorted so that they can be examined for contaminants once more, are parked, prior to said renewed examination for contaminants, e.g. for periods of at least 0.1, 1, 10, 100 h.

10. An apparatus (A) for examining filled containers (B, B1, B2, B3, B4, B5), which are filled with CO₂-containing products such as beer or lemonade, for contaminants such as glass fragments, comprising a measurement unit (M) which is capable of examining, e.g. by means of a camera, a container (B) to be examined for gas bubbles (G) in the product (P) / container (B) and/or for gas mists (N) in the product (P) / container (B) and for contaminants in the product (P) / container (B), **characterized in that,** the apparatus is configured for the case that if gas bubbles (G) are determined in the product (P) / in the container (B) and/or if gas mists (N) are determined in the product (P) / container (B), the container (B) can be sorted out and the container (B) can be fed back to the measurement unit (M) by a container guide (R) for renewed examination by means of the measurement unit (M) later on, or fed to some other alternative measurement unit.

11. The apparatus according to claim 10, **characterized in that** the apparatus (A) for examining filled containers (B) includes a container treatment unit (S) for a spin-stop process.

12. The apparatus according to one of the preceding claims 10 to 11, **characterized in that** the apparatus (A) for examining filled containers (B) includes a container parking station which is capable of accommodating filled containers (B).

13. The apparatus according to one of the preceding claims 10 to 12, **characterized in that** the measurement unit (M) includes an infrared camera (K).

## Revendications

1. Procédé pour inspecter des contenants remplis (B, B1, B2, B3, B4, B5), qui sont remplis de produits contenant du CO₂, tels que de la bière ou de la limonade, par rapport à des impuretés telles que des débris de verre, qui consiste à inspecter des bulles de gaz (G) dans le produit (P)/le contenant (B) et/ou un brouillard de gaz (N) dans le produit (P)/le contenant (B), par exemple au moyen d'une caméra, **caractérisé en ce que**, lorsque des bulles de gaz (G) sont détectées dans le produit (P)/le contenant (B) et/ou un brouillard de gaz (N) est détecté dans le produit (P)/le contenant (B), le contenant est trié avant de faire de nouveau l'objet d'une inspection d'impuretés ultérieurement.

2. Procédé selon la revendication 1, dans lequel l'inspection des contenants remplis (B, B1, B2, B3, B4, B5) concernant des impuretés comprend un procédé d'arrêt de rotation.

3. Procédé selon l'une quelconque des revendications précédentes, qui comprend la détection de bulles de gaz (G) dans le produit (P)/le contenant (B) qui survient en détectant un nombre minimum, par exemple au moins 10, 100 ou 1000, d'objets dans le produit (P) ou le contenant (B) avec des dimensions spatiales, par exemple de l'ordre de grandeur d'au moins 0,01, 0,1, 1, 10, 20 mm, et/ou lesdits objets sont détectés dans des positions de séjour prédéterminées.

4. Procédé selon l'une quelconque des revendications précédentes, qui comprend la détection d'un brouillard de gaz (N) dans le produit (P)/le contenant (B) qui survient en raison du fait que, sur une image de caméra du contenant (B) ou sur une image dérivée d'une ou plusieurs images de caméra, des zones étendues par exemple avec des dimensions spatiales d'au moins, par exemple, 0,1, 0,5, 1,0, 2,0 cm, une valeur de couleur et/ou d'échelle de gris et/ou une valeur de seuil de luminosité et/ou de contraste par rapport au produit (P)/à la teneur du contenant visé, par exemple, sont inférieures ou supérieures d'au moins 1, 10, 100 %, et/ou la détection d'un brouillard de gaz (N) dans le produit (P)/le contenant (8) survient en raison du fait que lesdites zones étendues présentent une variation/des variations inférieures, par exemple, à au moins 40, 30, 10 % par rapport à la valeur moyenne ou à la médiane de la distribution de la valeur de couleur et/ou d'échelle de gris et/ou des valeurs de luminosité et/ou de contraste à l'intérieur desdites zones étendues, avec des valeurs de couleur et/ou d'échelle de gris et/ou de luminosité et/ou de contraste du produit (P) différentes de la manière décrite ci-dessus dans leur forme, suivant le contour intérieur du contenant.

5. Procédé selon l'une quelconque des revendications précédentes, qui comprend la classification d'objets dans le contenant (B) sur la base de leur forme, par exemple des objets dans le contenant (B), comme des bulles de gaz (G), peuvent être identifiés si l'excentricité numérique de leur contour projeté dans l'image de caméra ou dans une image dérivée d'une ou plusieurs images de caméra n'est pas supérieure, par exemple, à au moins 0,1, 0,2, 0,3.

6. Procédé selon l'une quelconque des revendications précédentes, dans lequel la détection de bulles de gaz (G) dans le produit (P) et/ou d'un brouillard de gaz (N) dans le contenant (B) survient en utilisant des valeurs de seuil pour des différences de température, par exemple d'au moins 0,1, 0,5, 10, 20°C, pour distinguer/classer les différents objets/ différentes zones étendues dans le contenant (B).

7. Procédé selon l'une quelconque des revendications précédentes, qui comprend le marquage de contenants (8) remplis de produits contenant du CO₂ et dans lesquels une impureté a été détectée lors d'une première inspection, par exemple avec une empreinte/étiquette, et/ou leur évacuation à partir du processus de fabrication, et le passage sur des contenants (B) qui sont remplis de produits contenant du CO₂ et dans lesquels ni des impuretés ni des bulles de gaz (G) dans le produit (P)/le contenant (B) et/ou le brouillard de gaz (N) dans le produit (P)/le contenant (B) n'ont été constatés, sans objection, au processus de fabrication suivant.

8. Procédé selon l'une quelconque des revendications précédentes, qui comprend au final un déchargement des contenants (B) remplis de produits contenant du CO₂ à partir du processus de production lorsque des bulles de gaz (G) dans le produit (P)/le contenant (B) et/ou un brouillard de gaz (N) dans le produit (P)/le contenant (B) sont constatés lors d'une nouvelle inspection pour détecter des impuretés.

9. Procédé selon l'une des revendications précédentes, qui comprend le fait que des contenants (B) remplis, qui ont été triées afin de pouvoir être de nouveau inspectés par rapport à des impuretés, sont mis en stationnement avant une nouvelle inspection par rapport à des impuretés, par exemple pendant des périodes d'au moins 0,1, 10, 100 h.

10. Dispositif (A) pour inspecter des contenants remplis (B, B1, B2, B3, B4, B5) qui sont remplis de produits contenant du CO₂, comme par exemple de la bière ou de la limonade, pour détecter des impuretés, telles que des débris de verre, avec un dispositif de mesure (M), qui peut inspecter, par exemple avec une caméra, un contenant à inspecter (B) concernant des bulles de gaz (G) dans le produit (P)/le contenant (B) et/ou un brouillard de gaz (N) dans le produit (P)/le contenant (B) et pour inspecter des impuretés dans le produit (P)/le contenant (B), **caractérisé en ce que** le dispositif est configuré pour trié le contenant (B) lorsque des bulles de gaz (G) sont détectées dans le produit (P)/le contenant (B) et/ou un brouillard de gaz (N) est détecté dans le produit (P)/le contenant (B), et pour guider le contenant avec un guide de contenant (R), pour une nouvelle inspection par rapport à des impuretés ultérieurement au moyen du dispositif de mesure (M),vers le dispositif de mesure (M) ou vers un autre dispositif de mesure en variante.

11. Dispositif selon la revendication 10, **caractérisé en ce que** le dispositif (A) pour une inspection de contenants remplis (B) présente une unité de traitement de contenant (S) pour un procédé d'arrêt de rotation.

12. Dispositif selon l'une quelconque des précédentes revendications 10 à 11, **caractérisé en ce que** le dispositif (A) pour inspecter des contenants remplis (B) présente un poste de stationnement de contenants qui peut recevoir des contenants remplis (B).

13. Dispositif selon l'une des revendications précédentes 10 à 12, **caractérisé en ce que** le dispositif de mesure (M) comporte une caméra infrarouge (K).
